# EUROPÄISCHE PATENTANMELDUNG

(11) **EP 2 787 344 A1**
(43) Veröffentlichungstag der Anmeldung: **08.10.2014**
(21) Anmeldenummer: 14173229.7
(22) Anmeldetag: 01.06.2011
(51) Int. Cl.: G01N 33/36

(54) **Vorrichtung zur Bestimmung von Merkmalen eines Garns**

(30) Priorität: 09.06.2010 CH 924102010
(62) Teilanmeldung aus: 11731237.1
(71) Anmelder: Uster Technologies AG, 8610 Uster (CH)
(72) Erfinder: Die Erfindernennung liegt noch nicht vor
(74) Vertreter: Pliska, Pavel

(57) **Zusammenfassung**

Die Vorrichtung zur Bestimmung von Merkmalen, insbesondere der Haarigkeit, eines Garns beinhaltet eine Sensoreinrichtung zur Messung mindestens eines Parameters des Garns und eine Sensorabdeckung (49) zum Schutz der Sensoreinrichtung. Die Sensorabdeckung (49) weist einen Messspalt (44), durch welchen das Garn in seiner Längsrichtung (x) hindurch bewegbar ist, sowie einen Einlegeschlitz (45), durch welchen das Garn (9) in den Messspalt (44) einführbar ist, auf. Die Projektion des Einlegeschlitzes (45) in eine Ebene (yz) senkrecht zur Längsrichtung (x) ist gekrümmt. Dadurch wird verhindert, dass einerseits Licht von aussen in die Sensoreinrichtung gelangt und andererseits von einem Licht emittierenden Element der Sensoreinrichtung ausgesendetes Licht nach aussen gelangt.

## Beschreibung

### FACHGEBIET

Die vorliegende Erfindung liegt auf dem Gebiet der Qualitätskontrolle im Textillabor. Sie betrifft eine Vorrichtung zur Bestimmung von Merkmalen, insbesondere der Haarigkeit, eines Garns, gemäss den Oberbegriffen der unabhängigen Patentansprüche. Derartige Vorrichtungen kommen typischerweise in einem Textillabor zum Einsatz.

### STAND DER TECHNIK

Bei Stapelgarnen, die aus vielen Fasern gesponnen sind, sind einzelne Fasern nicht vollständig in den eigentlichen Garnkörper eingebunden. Sie ragen teilweise aus dem Garnkörper heraus, was man als Haarigkeit des Garns bezeichnet. Die Haarigkeit ist eine wichtige Garneigenschaft, weshalb die Textilindustrie ein grosses Interesse an ihrer zuverlässigen und reproduzierbaren Messung und Klassierung hat.

Vorrichtungen zur Bestimmung der Haarigkeit von Garnen sind bekannt, so z. B. das Gerät USTER^{®} *ZWEIGLE HAIRINESS TESTER 5* der Anmelderin, das z. B. im Anwendungshandbuch "USTER® ZWEIGLE HAIRINESS TESTER 5 Application Handbook", V1.0, September 2009, beschrieben ist, oder das Gerät USTER^{®} *TESTER 5-S800* der Anmelderin, das z. B. in der Broschüre "USTER® TESTER 5-S800 Brilliant Testing - Superior Analysis", August 2007, beschrieben ist. Auch die Schriften EP-0'271'728 A2, EP-1'621'872 A2, DE-198'18'069 A1 und DE-199'24'840 A1 offenbaren Haarigkeitsmessgeräte.

Die US-4,213,056 A offenbart eine Vorrichtung zur Prüfung von verflochtenen Multifilamentgarnen. Die Vorrichtung beinhaltet eine optische Sensoreinheit, in welcher sich ein Kontaktkörper befindet. Das zu prüfende Multifilamentgarn wird mit einem vorgegebenen Kontaktdruck über den Kontaktkörper geführt und dabei optisch abgetastet. Zur Einführung des Multifilamentgarns derart, dass es umschlingend um den Kontaktkörper geführt wird, ist in der Boden- und Deckenplatte der Sensoreinheit ein gekrümmter Einlegeschlitz vorgesehen.

Aus der EP-1'359'108 A1 ist eine Vorrichtung zur Detektion von Garnfehlern bekannt. Die Vorrichtung beinhaltet ein Gehäuse mit einer Durchgangsöffnung, durch welche das Garn durchläuft. Zum Einlegen des Garns in die Durchgangsöffnung dient ein Einlegeschlitz, der durch zwei ebene, zueinander parallele Wände des Gehäuses gebildet wird.

Die GB-916,520 beschreibt einen mechanischen Garnreiniger. Das Garn läuft in einem Spalt zwischen zwei Reinigerklingen hindurch. Dickstellen bleiben an den Reinigerklingen hängen, so dass Garn bei den Dickstellen reisst. Der Spalt zwischen den Reinigerklingen ist auf eine Seite hin trompetenförmig aufgeweitet. Um ein Passieren eines Knotens durch den Garnreiniger zu ermöglichen, wird das Garn vorübergehend mittels eines Hebels aus dem Spalt gehoben und nach Passieren des Knotens wieder durch den aufgeweiteten Teil in den Spalt eingelegt.

### DARSTELLUNG DER ERFINDUNG

Es ist eine Aufgabe der vorliegenden Erfindung, die bekannten Vorrichtungen zur Bestimmung von Merkmalen eines Garns weiter zu verbessern.

Diese und andere Aufgaben werden durch die erfindungsgemässe Vorrichtung, wie sie im ersten Patentanspruch definiert ist, gelöst. Eine vorteilhafte Ausführungsform ist im abhängigen Patentanspruch angegeben.

Die erfindungsgemässe Vorrichtung zur Bestimmung von Merkmalen eines Garns beinhaltet eine Sensoreinrichtung zur Messung mindestens eines Parameters des Garns und eine Sensorabdeckung zum Schutz der Sensoreinrichtung. Die Sensorabdeckung weist einen Messspalt, durch welchen das Garn in seiner Längsrichtung hindurch bewegbar ist, sowie einen Einlegeschlitz, durch welchen das Garn in den Messspalt einführbar ist, auf. Die Projektion des Einlegeschlitzes in eine Ebene senkrecht zur Längsrichtung ist gekrümmt. In einer bevorzugten Ausführung ist die Sensoreinrichtung eine optoelektronische Sensoreinrichtung, und der Einlegeschlitz ist derart gekrümmt, dass Licht von aussen nicht direkt auf das Garn und/oder auf ein lichtempfindliches Element der Sensoreinrichtung gelangt, und/oder dass von einem Licht emittierenden Element der Sensoreinrichtung ausgesendetes Licht nicht direkt und/oder über das Garn nach aussen gelangt. Dadurch wird einerseits die optoelektronische Sensoreinrichtung möglichst gut von unerwünschtem Fremdlicht abgeschattet und das Messsignal verbessert. Andererseits wird die Arbeitssicherheit erhöht, weil Personen in der Umgebung der Vorrichtung nicht durch austretendes Licht verletzt werden können. Mit dem Begriff "Licht" ist in dieser Schrift nicht nur der sichtbare Bereich (VIS) des elektromagnetischen Spektrums gemeint, sondern auch die daran angrenzenden Bereiche Ultraviolett (UV) und Infrarot (IR).

In einer ersten, nicht beanspruchten Ausführungsform beinhaltet die Vorrichtung zur Bestimmung von Merkmalen eines Garns ein Gehäuse und eine in oder an dem Gehäuse angebrachte Sensoreinrichtung zur Messung mindestens eines Parameters des Garns. Das Gehäuse besteht im Wesentlichen aus einer Frontplatte, einer Rückplatte und einem zwischen der Frontplatte und der Rückplatte liegenden Rahmen. Der Rahmen ist einstückig und gesondert von der Frontplatte sowie der Rückplatte gefertigt. Der Rahmen ist vorzugsweise aus einem gebogenen Metallblech gefertigt. Die Frontplatte und die Rückplatte können als ebene Platten ausgebildet und mittels Schraubverbindungen an Laschen, die am Rahmen nach innen gebogen sind, befestigt sein. In dieser ersten Ausführungsform hat das Gehäuse einen besonders einfachen Aufbau.

In einer zweiten, nicht beanspruchten Ausführungsform beinhaltet die Vorrichtung zur Bestimmung von Merkmalen eines Garns ein Gehäuse mit einer im Wesentlichen senkrecht auf einer Unterlage stehenden Frontplatte, eine in oder an dem Gehäuse angebrachte Sensoreinrichtung zur Messung mindestens eines Parameters des Garns und weitere Bauteile. Die Frontplatte des Gehäuses dient als Trägerelement für die Sensoreinrichtung und mehrere weitere Bauteile. In einer bevorzugten Ausführung sind die mehreren weiteren Bauteile aus der Menge ausgewählt, welche die folgenden Elemente enthält: Führungselemente zur Garnführung, eine Sensorabdeckung für die Sensoreinrichtung, eine elektronische Schaltung zur Steuerung von Teilen der Vorrichtung und/oder zur Auswertung von Signalen der Sensoreinrichtung, eine Garnfördereinrichtung, eine Rollenabdeckung für die Garnfördereinrichtung, eine Antriebseinrichtung zum Antreiben der Garnfördereinrichtung, eine Steuerung der Antriebseinrichtung, eine Changiereinrichtung, eine Garnabsaugeinrichtung und einen Andruckzylinder für eine Garnförderrolle. Die Sensoreinrichtung und die mehreren weiteren Bauteile können unmittelbar an der Frontplatte und/oder mittelbar an Winkelprofilen, die ihrerseits and der Frontplatte befestigt sind, angebracht sein. Auch diese zweite Ausführungsform trägt zu einem einfachen, übersichtlichen Aufbau der Vorrichtung und zu deren einfachen Wartbarkeit bei.

In einer dritten, nicht beanspruchten Ausführungsform beinhaltet die Vorrichtung zur Bestimmung von Merkmalen eines Garns eine Sensoreinrichtung zur Messung mindestens eines Parameters des Garns und eine Garnfördereinrichtung zur Förderung des Garns durch die Sensoreinrichtung. Die Garnfördereinrichtung ist als Rollenlieferwerk mit zwei zusammen wirkenden Förderrollen ausgebildet. Achsen der Förderrollen liegen parallel zueinander und weisen einen veränderbaren Abstand voneinander auf, und mindestens eine der Förderrollen ist angetrieben. Die Vorrichtung beinhaltet ferner einen Endlagensensor oder Endlagenschalter, welcher den Antrieb der angetriebenen Förderrolle unterbricht, wenn der Abstand zwischen den beiden Achsen einen vorgegebenen Schwellenwert überschritten hat. Diese vierte Ausführungsform bietet somit einen wirksamen Schutz vor unerwünschter Wickelbildung und Einklemmen von Fingern, Haaren, Kleidungs- oder Schmuckstücken.

In einer vierten, nicht beanspruchten Ausführungsform beinhaltet die Vorrichtung zur Bestimmung von Merkmalen eines Garns eine Sensoreinrichtung zur Messung mindestens eines Parameters des Garns und eine Garnfördereinrichtung zur Förderung des Garns durch die Sensoreinrichtung. Die Garnfördereinrichtung ist als Rollenlieferwerk mit zwei zusammen wirkenden Förderrollen ausgebildet ist, wobei Achsen der Förderrollen parallel zueinander liegen. Die Vorrichtung beinhaltet ferner eine Changiereinrichtung, welche im Bereich der Garnfördereinrichtung während der Garnförderung die Lage des Garns in Richtung der Achsen der Förderrollen zeitlich ändert. Zu diesem Zweck kann die Changiereinrichtung ein als Garnführungsöse ausgebildetes Changierelement beinhalten, welches in Richtung der Achsen der Förderrollen hin und her bewegbar ist, bspw. durch einen pneumatischen Changierzylinder. Mit dieser fünften Ausführungsform werden die Manteloberflächen der Förderrollen in Achsrichtung gleichmässig, statt nur an einer Position, beansprucht, was ihre Lebensdauer erhöht.

In einer fünften, nicht beanspruchten Ausführungsform beinhaltet die Vorrichtung zur Bestimmung von Merkmalen eines Garns eine Sensoreinrichtung zur Messung mindestens eines Parameters des Garns und eine Garnfördereinrichtung zur Förderung des Garns durch die Sensoreinrichtung. Die Garnfördereinrichtung ist als Rollenlieferwerk mit zwei zusammen wirkenden Förderrollen ausgebildet, wobei mindestens eine der Förderrollen durch eine Antriebseinrichtung antreibbar ist. Die mindestens eine antreibbare Förderrolle ist direkt durch die Antriebseinrichtung antreibbar. Dies macht die Vorrichtung wiederum einfach und kostengünstig. Zwischen der Antriebseinrichtung und der mindestens einen antreibbaren Förderrolle kann eine flexible Kupplung angebracht sein, die vorzugsweise als Spiralkupplung oder Balgkupplung ausgebildet ist. Dadurch wird ein gleichmässiger Garnlauf gewährleistet.

Die oben beschriebenen Ausführungsformen können beliebig miteinander kombiniert werden. Durch derartige Kombinationen ergeben sich weitere, besonders vorteilhafte Ausführungsformen.

Die erfindungsgemässe Vorrichtung kann zur Bestimmung von verschiedenen Merkmalen eines Garns verwendet werden. Sie kann zur Bestimmung nur eines einzigen Merkmals oder von mehreren unterschiedlichen Merkmalen geeignet sein. Eine besonders vorteilhafte Verwendung der erfindungsgemässen Vorrichtung besteht in der optoelektronischen Messung der Haarigkeit des Garns.

### AUFZÄHLUNG DER ZEICHNUNGEN

Nachfolgend werden verschiedene Aspekte der erfindungsgemässen Vorrichtung anhand der Zeichnungen detailliert erläutert.
- Figur 1: zeigt eine erfindungsgemässe Vorrichtung in einer perspektivischen Ansicht.
- Figur 2: zeigt den Aufbau eines Gehäuses der erfindungsgemässen Vorrichtung.
- Figur 3: zeigt eine Frontplatte der erfindungsgemässen Vorrichtung (a) in einer Vorderansicht und (b) in einer Rückansicht.
- Figur 4: zeigt eine Sensorabdeckung der erfindungsgemässen Vorrichtung in einer perspektivischen Ansicht.
- Figur 5: zeigen Förderrollen und eine Changiereinrichtung der erfindungsgemässen Vorrichtung in perspektivischen Ansichten (a) von oben und (b) von der Seite.
- Figur 6: zeigt eine Kupplung für den Garnantrieb der erfindungsgemässen Vorrichtung in einer Seitenansicht.

### AUSFÜHRUNG DER ERFINDUNG

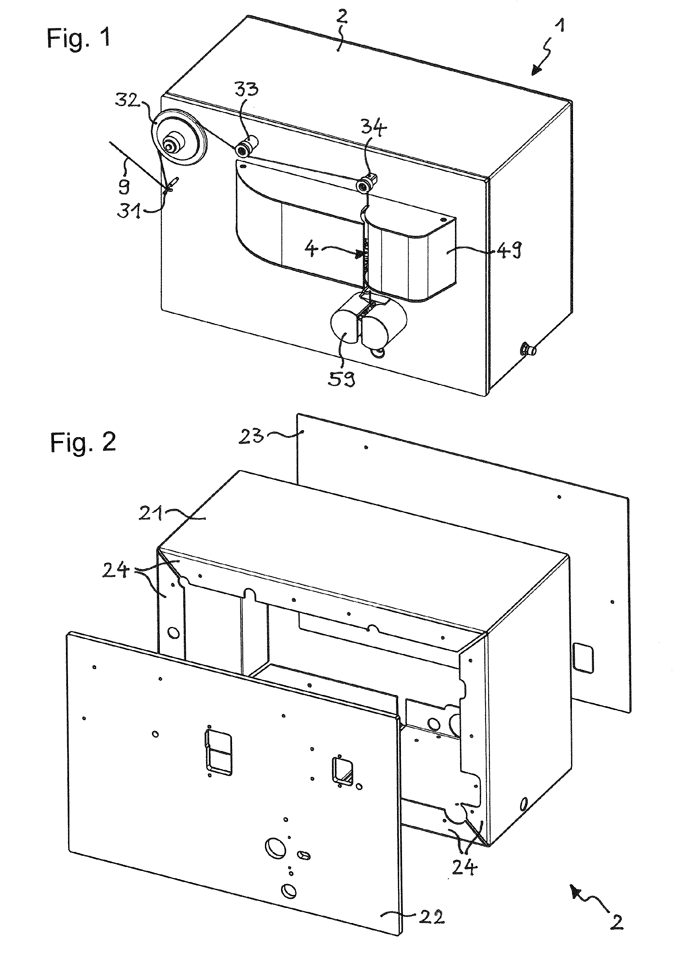

**Figur 1** zeigt von aussen eine erfindungsgemässe Vorrichtung 1 zur Bestimmung von Merkmalen eines Garns 9. Man erkennt in dieser Darstellung ein im Wesentlichen quaderförmiges Gehäuse 2, verschiedene Garnführungselemente 31-34 zur Führung des Garns 9, eine Sensorabdeckung 49 für eine Garnsensoreinrichtung 4 und eine Rollenabdeckung 59 für eine Garnfördereinrichtung 5. Die Garnsensoreinrichtung 4 misst mindestens einen Parameter des Garns 9, z. B. die Länge von Fasern ("Haaren"), die vom Garn 9 abstehen.

In **Figur 2** ist der besonders einfache Aufbau des Gehäuses 2 der Vorrichtung 1 dargestellt. Das Gehäuse 2 besteht im Wesentlichen aus einem Rahmen 21, einer Frontplatte 22 und einer Rückplatte 23. Der Rahmen 21 ist einstückig gefertigt, vorzugsweise indem ein Metallblech gebogen und an den Enden zusammengeschweisst wird, so dass im Wesentlichen eine Mantelfläche eines Quaders entsteht. Für den Rahmen 21 kann z. B. ca. 2 mm dickes Stahlblech verwendet werden. Die Frontplatte 22 und die Rückplatte 23 sind jeweils als ebene Metallplatten ausgebildet. Sie können z. B. aus ca. 2-16 mm dickem Stahl oder Aluminium bestehen. Die Befestigung der Frontplatte 22 und der Rückplatte 23 am Rahmen 21 kann mittels Schraubverbindungen an Laschen 24, die am Rahmen 21 nach innen gebogen sind, erfolgen.

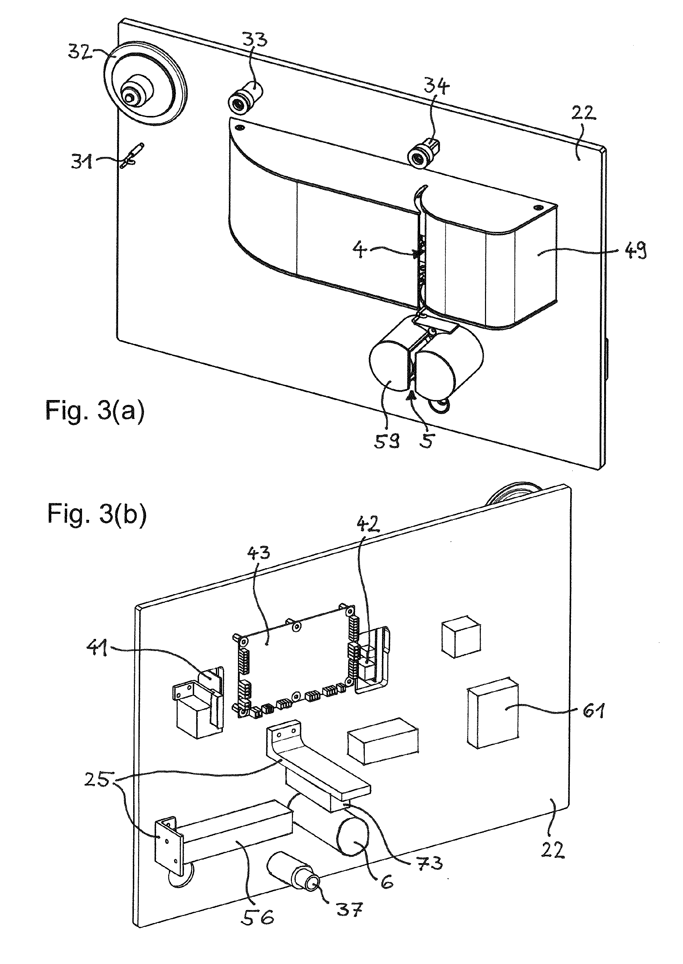

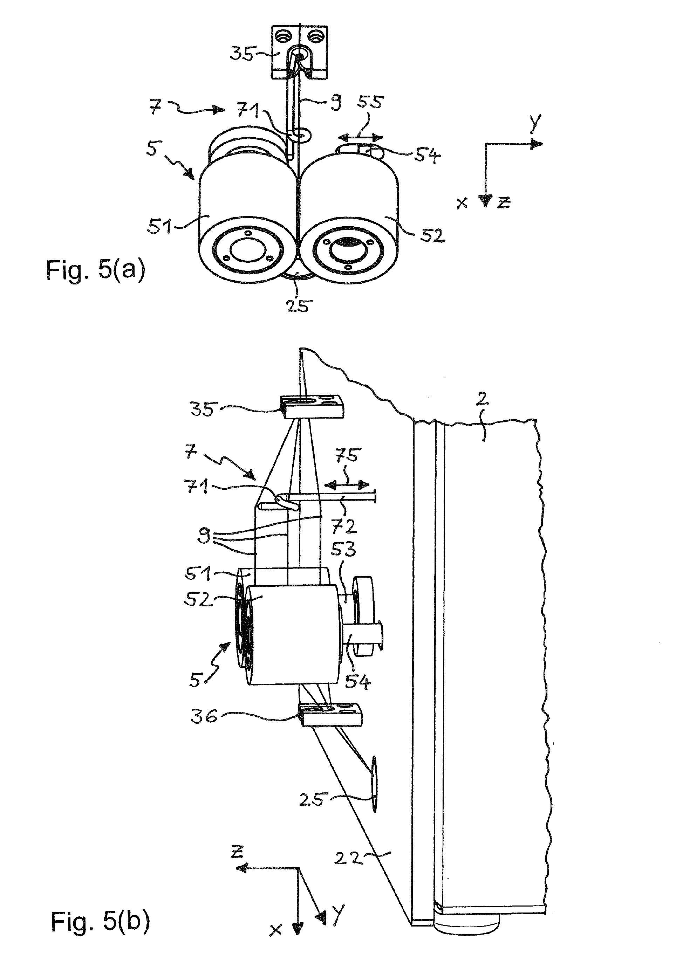

**Figur 3** zeigt die Frontplatte 22 (a) in einer Vorderansicht und (b) in einer Rückansicht. Die Frontplatte 22 dient als Trägerelement für möglichst viele Bauteile der Vorrichtung 1. Im Beispiel von Figur 3 trägt die Frontplatte 22 auf ihrer Vorderseite (Fig. 3(a)) die Sensorabdeckung 49, die (von der Sensorabdeckung 49 abgedeckte und in Fig. 3(b) teilweise sichtbare) Sensoreinrichtung 4, die bspw. einen Lichtsender 41 und einen Lichtempfänger 42 beinhalten kann, die Rollenabdeckung 59, die (von der Rollenabdeckung 59 abgedeckten) Garnfördereinrichtung 5 und die Garnführungselemente 31-34. Auf der Rückseite (Fig. 3(b)) der Frontplatte 22 sind z. B. eine Leiterplatte 43 mit einer elektronischen Schaltung zur Steuerung von Teilen der Vorrichtung 1 und/oder zur Auswertung der Sensorsignale, eine Antriebssteuerung 61, ein Changierzylinder 73, eine Antriebseinrichtung 6, eine Garnabsaugeinrichtung 37 und einen Andruckzylinder 56 für eine Anpressrolle 52 (vgl. Fig. 5). Diese und/oder andere Bauteile können unmittelbar an der Frontplatte 22 oder mittelbar an Winkelprofilen 25, die ihrerseits and der Frontplatte 22 befestigt sind, angebracht sein.

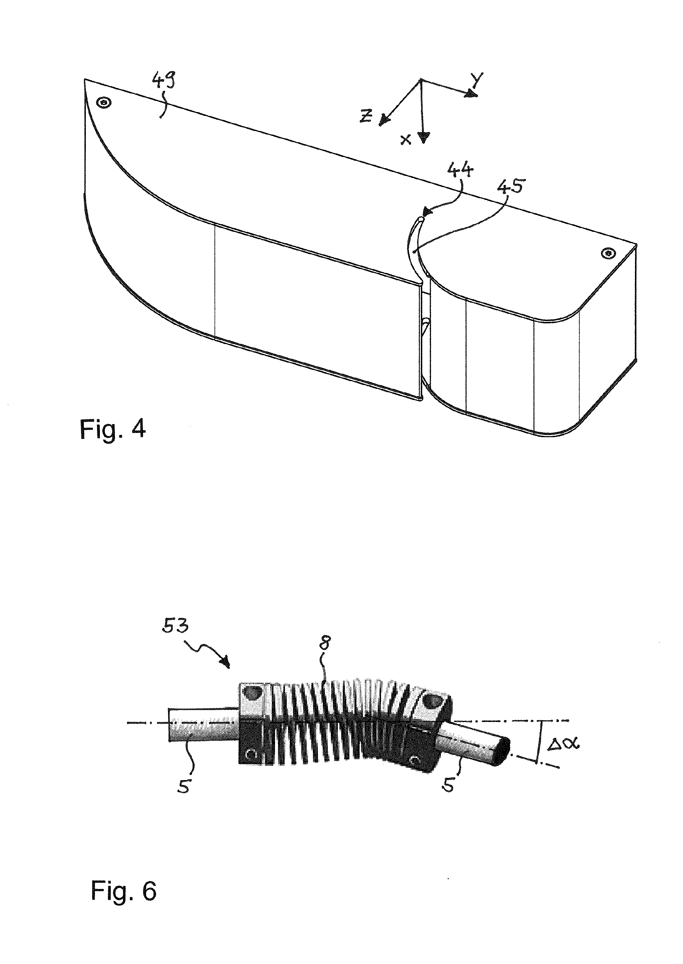

Die Sensorabdeckung 49 der Vorrichtung 1 ist in **Figur 4** dargestellt. Die Sensorabdeckung 49 schützt die Sensoreinrichtung 4 der Vorrichtung 1 (vgl. Fig. 1) vor mechanischen Einwirkungen, vor Verschmutzung und im Fall einer optoelektronischen Sensoreinrichtung 4 vor Fremdlichteinfall. Im letzteren Fall ist die Sensorabdeckung 49 in demjenigen Spektralbereich, in welchem die optoelektronische Sensoreinrichtung 4 lichtempfindlich ist, lichtundurchlässig. Die Sensorabdeckung 49 weist einen Messspalt 44 auf, durch welchen das (in Fig. 4 nicht dargestellte) Garn 9 in seiner Längsrichtung x hindurch bewegt wird, sowie einen Einlegeschlitz 45, durch welchen das Garn 9 in den Messspalt 44 eingeführt werden kann. Der Messspalt 44 und der Einlegeschlitz 45 verlaufen parallel zur Längsrichtung x. Gemäss der Erfindung ist die Projektion des Einlegeschlitzes 45 in die Ebene (yz) senkrecht zur Längsrichtung x gekrümmt. Eine solche Krümmung bringt mehrere Vorteile mit sich. Einerseits verhindert sie, dass durch den Einlegeschlitz 45 Licht von aussen direkt auf das Garn 9 und/oder auf ein lichtempfindliches Element 42 (vgl. Fig. 3(b)) der Sensoreinrichtung 4 gelangt. Dadurch wird die optoelektronische Sensoreinrichtung 4 möglichst gut von unerwünschtem Fremdlicht abgeschattet und das Messsignal verbessert. Andererseits verhindert die Krümmung auch, dass vom Lichtsender 42 ausgesendetes Licht direkt und/oder über das Garn 9 nach aussen gelangt und möglicherweise Verletzungen bei Personen verursacht. Somit wird die Arbeitssicherheit erhöht.

**Figur 5** zeigt eine Garnfördereinrichtung 5 und eine Changiereinrichtung 7 der erfindungsgemässen Vorrichtung 1 (a) von oben und (b) von der Seite. Die Garnfördereinrichtung 5 ist als Rollenlieferwerk mit zwei zusammen wirkenden Förderrollen 51, 52 ausgebildet. Eine erste, aktive Antriebsrolle 51 ist zur Rotation angetrieben. Der Antrieb kann durch eine Antriebseinrichtung 6 (siehe Fig. 3(b)), z. B. einen Elektromotor, über eine Welle 53 erfolgen. Eine zweite, passive Anpressrolle 52 ist eine antriebslose, um eine Achse 54 drehbare Rolle. Die Manteloberflächen der beiden Förderrollen 51, 52 sind zur Kontaktierung und Mitnahme des zu fördernden Garns 9 geeignet und bestehen z. B. aus Metall oder einem Elastomer. Die Welle 53 und die Achse 54 der beiden Förderrollen 51, 52 sind parallel zueinander. Die beiden Manteloberflächen berühren einander, wenn kein Garn 9 eingelegt ist; andernfalls klemmen sie das Garn 9 zwischen einander und fördern es in Längsrichtung x durch die (in Fig. 5 nicht eingezeichnete) Sensoreinrichtung 4.

Die Achse 54 der Anpressrolle 52 ist in Richtung y senkrecht zur Achsrichtung z und senkrecht zur Garnlängsrichtung x in einem kleinen Bereich von bspw. 3 mm beweglich, was in Fig. 5(a) durch einen Doppelpfeil 55 angedeutet ist. Sie wird mit einer Kraft beaufschlagt, welche die Anpressrolle 52 gegen die Antriebsrolle 51 drückt, bspw. mittels eines pneumatischen Andruckzylinders 56 (siehe Fig. 3(b)). Erreicht die Achse 54 eine vorgegebene, äussere Position, so stellt ein (nicht eingezeichneter) Endlagensensor oder Endlagenschalter die Antriebseinrichtung 6 automatisch ab. Dadurch wird erstens sichergestellt, dass die Antriebseinrichtung 6 bei offener Stellung der Förderrollen 51, 52, z. B. beim Einlegen des Garns 9, nicht läuft. Zweitens wird so die Bildung eines Wickels verhindert. Von einem Wickel spricht man, wenn sich das Garn 9 unerwünschterweise auf einer der Förderrollen 51, 52 aufzuwickeln beginnt. Ist dies der Fall, so vergrössert das aufgewickelte Garn 9 den Abstand zwischen der Welle 53 und der Achse 54, so dass schliesslich der Endlagesensor anspricht und die Antriebseinrichtung 6 abstellt. Ebenso wird die Antriebseinrichtung abgestellt, wenn Finger, Haaren, Kleidungs- oder Schmuckstücke einer Bedienungsperson zwischen die Förderrollen 51, 52 gelangen. Drittens werden also Unfälle und Verletzungen vermieden.

Ein weiterer Aspekt der Vorrichtung 1 betrifft die Garnchangierung. Darunter versteht man eine zeitliche Änderung der Lage des Garns 9 in Richtung z der Welle 53 und der Achse 54, um die Manteloberflächen der Förderrollen 51, 52 in Achsrichtung z gleichmässig, statt nur an einer Position, zu beanspruchen. Die Garnchangierung wird erfindungsgemäss erzielt, indem im Bereich der Fördereinrichtung 5 die Lage des Garns 9 in Achsrichtung z mittels einer ein Changierelement 71 beinhaltenden Changiereinrichtung 7 verändert wird. In Fig. 5(b) ist die Changierbewegung mit einem Doppelpfeil 75 angedeutet, und das Garn 9 ist in drei verschiedenen Lagen eingezeichnet. Im hier diskutierten Ausführungsbeispiel ist das Changierelement 71 als Garnführungsöse, die am Ende eines in Achsrichtung z beweglichen Stabes 72 angebracht ist, ausgeführt. Der Stab 72 und damit die Garnführungsöse 71 wird während der Garnförderung mittels eines pneumatischen Changierzylinders 73 (siehe Fig. 3(b)) langsam in Achsrichtung z hin und her bewegt. "Langsam" heisst in diesem Zusammenhang, dass während eines Changierzyklus mehrere Meter Garn durch die Fördereinrichtung 5 gefördert werden, z. B. 2-20 m und vorzugsweise 6-12 m. Die Changiereinrichtung 7 befindet sich stromaufwärts bezüglich der Förderrollen 51, 52. Ein erstes ortsfestes Garnführungselement 35 ist zwischen der Changiereinrichtung 7 und der Sensoreinrichtung 4 angebracht und sorgt für einen stabilen, stationären Garnlauf durch die Sensoreinrichtung 4. Vorzugsweise ist stromabwärts bezüglich der Förderrollen 51, 52 ein zweites ortsfestes Garnführungselement 36 angebracht. Im Bereich der Sensoreinrichtung 4 können auch weitere ortsfeste Garnführungselemente vorgesehen sein, z. B. auf der Sensorabdeckung 49. Mindestens eines der ortsfesten Garnführungselemente 35, 36 kann als Fallenöse ausgebildet sein, in welche das Einlegen des Garns 9 von vom, d. h. durch den Einlegeschlitz, 45, einfach ist, ein Herausziehen in umgekehrter Richtung jedoch schwierig. Das durch die Sensoreinrichtung 4 geförderte Garn 9 wird mittels der Garnabsaugeinrichtung 37 (siehe Fig. 3(b)) durch eine Absaugöffnung 25 in der Frontplatte 22 in einen (nicht dargestellten) Abfallbehälter abgesaugt.

Die Antriebsrolle 51 wird von der Antriebseinrichtung 6 direkt angetrieben, d. h. ohne die sonst üblichen Übertragungsriemen oder Getriebe. Dies macht die Vorrichtung 1 besonders einfach und kostengünstig. Es ist vorteilhaft, an der Welle 53 zwischen der Antriebseinrichtung 6 und der Antriebsrolle 51 eine flexible Kupplung 8 anzubringen, welche einen möglichen Ortsversatz und/oder Winkelversatz Δα zwischen der Antriebwelle 57 und der Abtriebwelle 58 ausgleicht und so einen gleichmässigen Garnlauf gewährleistet. Eine solche flexible Kupplung 8 ist in Figur 6 dargestellt. Vorzugsweise kommt eine Spiralkupplung oder eine Balgkupplung aus Metall oder Kunststoff zum Einsatz.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören, wie sie in den unabhängigen Patentansprüchen definiert ist.

### BEZUGSZEICHENLISTE

- 1: Vorrichtung

- 2: Gehäuse
- 21: Rahmen
- 22: Frontplatte
- 23: Rückplatte
- 24: Laschen
- 25: Absaugöffnung

- 31-34: Garnführungselemente
- 35, 36: ortsfeste Garnführungselemente

- 4: Sensoreinrichtung
- 41: Lichtsender
- 42: Lichtempfänger
- 43: Leiterplatte
- 44: Messspalt
- 45: Einlegeschlitz
- 49: Sensorabdeckung
- 5: Garnfördereinrichtung
- 51: Antriebsrolle
- 52: Anpressrolle
- 53: Welle der Antriebsrolle
- 54: Achse der Anpressrolle
- 55: Bewegung der Achse
- 56: Andruckzylinder für Anpressrolle
- 57: Antriebswelle
- 58: Abtriebswelle
- 59: Rollenabdeckung

- 6: Antriebseinrichtung

- 7: Changiereinrichtung
- 71: Changierelement
- 72: Stab
- 73: Changierzylinder
- 75: Changierbewegung

- 8: flexible Kupplung

- 9: Garn

- x: Garnlängsrichtung, Garnlaufrichtung
- y: Richtung senkrecht zur Garnlängsrichtung, parallel zur Frontplatte
- z: Richtung senkrecht zur Garnlängsrichtung, senkrecht zur Frontplatte

## Patentansprüche

1. Vorrichtung (1) zur Bestimmung von Merkmalen eines Garns (9), beinhaltend eine Sensoreinrichtung (4) zur Messung mindestens eines Parameters des Garns (9) und
eine Sensorabdeckung (49) zum Schutz der Sensoreinrichtung (4), wobei die Sensorabdeckung (49) einen Messspalt (44), durch welchen das Garn (9) in seiner Längsrichtung (x) hindurch bewegbar ist, sowie einen Einlegeschlitz (45), durch welchen das Garn (9) in den Messspalt (44) einführbar ist, aufweist,
**dadurch gekennzeichnet,**
**dass** die Projektion des Einlegeschlitzes (45) in eine Ebene (yz) senkrecht zur Längsrichtung (x) gekrümmt ist.

2. Vorrichtung (1) nach Anspruch 1, wobei die Sensoreinrichtung (4) eine optoelektronische Sensoreinrichtung (4) ist und der Einlegeschlitz (45) derart gekrümmt ist, dass Licht von aussen nicht direkt auf das Garn (9) und/oder auf ein lichtempfindliches Element (42) der Sensoreinrichtung (4) gelangt, und/oder dass von einem Licht emittierenden Element (41) der Sensoreinrichtung (4) ausgesendetes Licht nicht direkt und/oder über das Garn nach aussen gelangt.

3. Verwendung der Vorrichtung (1) nach einem der vorangehenden Ansprüche zur Bestimmung der Haarigkeit des Garns (9).
